# EUROPEAN PATENT SPECIFICATION

(11) **EP 2 169 395 B1**
(45) Date of publication and mention of the grant of the patent: **13.06.2018**
(21) Application number: 09169288.9
(22) Date of filing: 02.09.2009
(51) Int. Cl.: G01N 27/419

(54) **Exhaust gas sensing system and method for determining concentrations of exhaust gas constituents**
Abgassensorsystem und Verfahren zur Bestimmung von Abgaskonzentrationenbestandteilen
Système de détection de gaz d'échappement et procédé pour déterminer les concentrations des constituants de gaz d'échappement

(30) Priority: 29.09.2008 US 240354; 18.12.2008 US 337784
(43) Date of publication of application: 31.03.2010
(73) Proprietor: Delphi Technologies, Inc., Troy MI 48007 (US)
(72) Inventor: WANG, Da Yu, Troy, MI 48084 (US); YAU, Sheng, Macomb, MI 48044 (US); CABUSH, David D., Howell, MI 48843 (US); SYMONS, Walter Thomas, Grand Blanc, MI 48439 (US); FARHAT, Robert Jerome, Grosse Pointe Woods, MI 48236 (US)
(74) Representative: Delphi France SAS

(56) References cited:
- EP-A2- 0 994 346
- US-A1- 2004 118 703
- US-A1- 2007 080 074
- US-B1- 6 274 016
- US-B2- 6 607 643

## Description

### TECHNICAL FIELD

The invention relates to exhaust gas sensing system and method for determining concentrations of exhaust gas constituents.

### BACKGROUND OF THE INVENTION

A nitrogen oxides (NOₓ) sensor has been developed that detects NOₓ concentrations. However, the NOₓ sensor is not capable of directly determining nitrogen dioxide (NO₂) concentrations. Further, the NOₓ sensor may not be able to determine NOₓ concentrations in exhaust gases when the exhaust gases have ammonia (NH₃) therein.

Accordingly, the inventors herein have recognized a need for an improved exhaust gas sensor that minimizes and/or eliminates the above-mentioned deficiencies.

### SUMMARY OF THE INVENTION

An exhaust gas sensing system in accordance with an exemplary embodiment is provided. The exhaust gas sensing system includes a NH₃ sensing cell configured to generate a first voltage indicative of a combination of a NH₃ concentration and a NO₂ concentration in exhaust gases communicating with the NH₃ sensing cell. The exhaust gas sensing system further includes a NO₂ sensing cell configured to generate a second voltage indicative of a NO₂ concentration in exhaust gases communicating with the NO₂ sensing cell. The exhaust gas sensing system further includes a NOₓ pumping cell configured to generate an electrical current indicative of combination of a NO concentration, a NO₂ concentration, and a NH₃ concentration in exhaust gases communicating with the NOₓ pumping cell. The exhaust gas sensing system further includes a current sensor configured to measure the electrical current flowing through the NOₓ pumping cell and to output a current measurement signal indicative of a magnitude of the electrical current. The exhaust gas sensing system further includes a computer configured to receive the first voltage, the second voltage, and the current measurement signal. The computer is further configured to determine a NO₂ concentration value indicative of the NO₂ concentration in the exhaust gases communicating with the NO₂ sensing cell based on the second voltage. The computer is further configured to determine a NH₃ concentration value indicative of the NH₃ concentration in the exhaust gases communicating with the NH₃ sensing cell based on the first voltage and the NO₂ concentration value. The computer is further configured to determine a nitrogen monoxide (NO) concentration value indicative of a NO concentration in the exhaust gases communicating with the NOₓ pumping cell based on the current measurement signal, the NO₂ concentration value, and the NH₃ concentration value. The computer is further configured to determine a NOₓ concentration value based on the NO concentration value and the NO₂ concentration value. The computer is further configured to store the NO₂ concentration value, the NH₃ concentration value, the NO concentration value, and the NOₓ concentration value in a memory device.

A method for determining concentrations of exhaust gas constituents in accordance with another exemplary embodiment is provided. The method includes generating a first voltage utilizing a NH₃ sensing cell that is indicative of a combination of a NH₃ concentration and a NO₂ concentration in exhaust gases communicating with the NH₃ sensing cell. The method further includes generating a second voltage utilizing a NO₂ sensing cell that is indicative of a NO₂ concentration in exhaust gases communicating with the NO₂ sensing cell. The method further includes generating an electrical current utilizing a NOₓ pumping cell that is indicative of combination of a NO concentration, a NO₂ concentration, and a NH₃ concentration in exhaust gases communicating with the NOₓ pumping cell. The method further includes measuring the electrical current flowing through the NOₓ pumping cell utilizing a current sensor and outputting a current measurement signal from the current sensor indicative of a magnitude of the electrical current. The method further includes receiving the first voltage, the second voltage, and the current measurement signal at a computer. The method further includes determining a NO₂ concentration value indicative of the NO₂ concentration in the exhaust gases communicating with the NO₂ sensing cell based on the second voltage, utilizing the computer. The method further includes determining a NH₃ concentration value indicative of the NH₃ concentration in the exhaust gases communicating with the NH₃ sensing cell based on the first voltage and the NO₂ concentration value, utilizing the computer. The method further includes determining a NO concentration value indicative of a NO concentration in the exhaust gases communicating with the NOₓ pumping cell based on the current measurement signal, the NO₂ concentration value, and the NH₃ concentration value, utilizing the computer. The method further includes determining a NOₓ concentration value based on the NO concentration value and the NO₂ concentration value, utilizing the computer. The method further includes storing the NO₂ concentration value, the NH₃ concentration value, the NO concentration value, and the NOₓ concentration value in a memory device, utilizing the computer.

### BRIEF DESCRIPTION OF THE DRAWINGS

Figure 1 is a schematic of a vehicle having first and second exhaust gas sensing systems in accordance with exemplary embodiments;
Figure 2 is a schematic of the first exhaust gas sensing system of Figure 1;
Figures 3 and 4 are flowcharts of a method for determining concentrations of exhaust gas constituents in accordance with another exemplary embodiment;
Figure 5 is a schematic of a first exhaust gas sensor utilized in the second exhaust gas sensing system of Figure 1; and
Figure 6 is a schematic of a second exhaust gas sensor utilized in the second exhaust gas sensing system of Figure 1.

### DESCRIPTION OF THE PREFERRED EMBODIMENTS

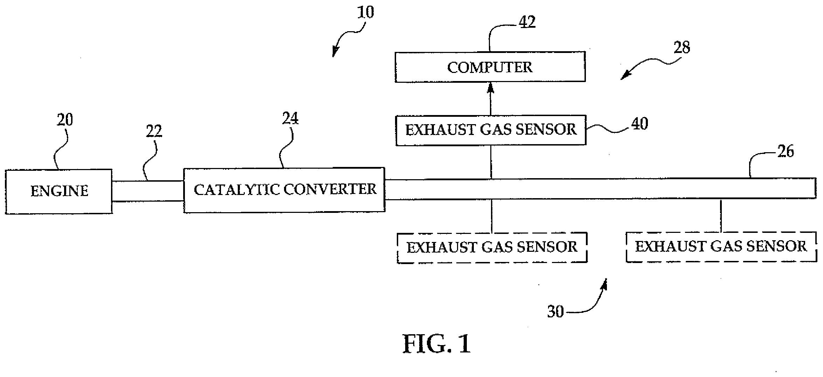

Referring to Figure 1, a vehicle 10 is illustrated. The vehicle 10 includes an engine 20, an exhaust pipe 22, a catalytic converter 24, an exhaust pipe 26, and exhaust gas sensing systems 28, 30. The engine 20 emits exhaust gases that are routed through the exhaust pipe 22 and the catalytic converter 24 to the exhaust pipe 26. The exhaust gas sensing systems 28, 30 communicate with the exhaust pipe 26 to determine concentrations of exhaust gas constituents in the exhaust gases. In particular, the exhaust gas sensing systems 28, 30 can accurately determine a NO₂ concentration, a NH₃ concentration, a NO concentration, and a NOₓ concentration in the exhaust gases. The sensors of the exhaust gas sensing systems 28 and 30 can also be positioned in other parts of the exhaust system. For example, the sensors can be positioned immediately downstream of the engine 20 but upstream of the catalytic converter 24, or inside the catalytic converter 24.

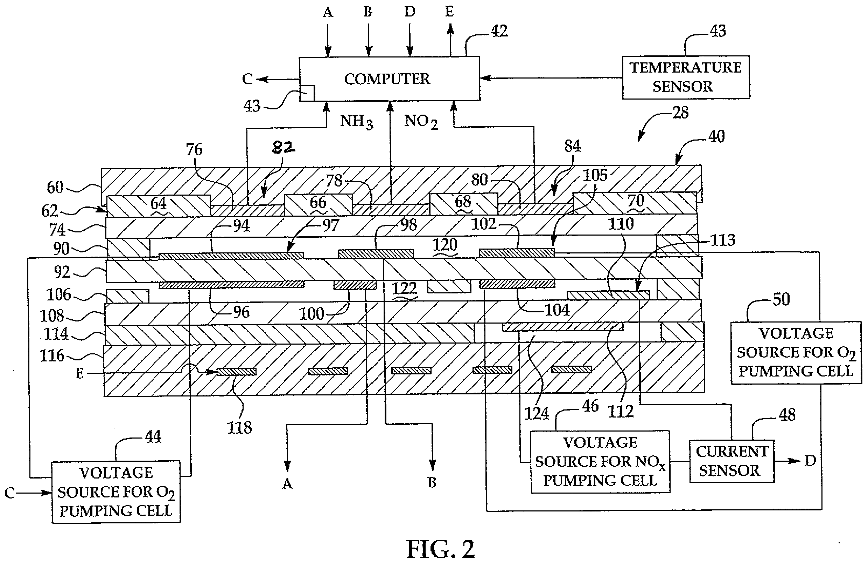

Referring to Figure 2, the exhaust gas sensing system 28 includes an exhaust gas sensor 40, a temperature sensor 43, a voltage source 44, a voltage source 46, a current sensor 48, and a voltage source 50.

The exhaust gas sensor 40 communicates with the exhaust gases in the exhaust pipe 26 to generate signals that are either directly or indirectly indicative of concentrations of NO₂, NH₃, NO, and NOₓ in the exhaust gases in the exhaust pipe 26. The exhaust gas sensor 40 includes a porous protection layer 60, an alumina layer 62, a zirconia layer 74, a NH₃ sensing electrode 76, a reference electrode 78, a NO₂ sensing electrode 80, an alumina layer 90, a zirconia layer 92, pump electrodes 94, 96, an O₂ reference electrode 98, an oxygen sensing electrode 100, pump electrodes 102, 104, an alumina layer 106, a zirconia layer 108, a NO₂ sensing electrode 80, a NOₓ sensing electrode 110, a reference electrode 112, alumina layers 114, 116, and a heater coil 118.

The porous protection layer 60 is configured to allow exhaust gases to diffuse therethrough to contact the NH₃ sensing electrode 76, the reference electrode 78, and the NO₂ sensing electrode 80. In one exemplary embodiment, the porous protection layer 60 is constructed from porous alumina. As shown, the layer 60 is disposed at least in part on a first side of the alumina layer 62.

A second side of the alumina layer 62 is disposed on a first side of the zirconia layer 74. The alumina layer 62 includes portions 64, 66, 68, 70 disposed apart from one another that form gaps therebetween. The NH₃ sensing electrode 76 is disposed in a gap between the portions 64, 66 of the alumina layer 62 and is further disposed between the layer 60 and the zirconia layer 74. In one exemplary embodiment, the NH₃ sensing electrode 76 is constructed of BiVO₄ doped with 0-20 mole % of MgO, 0-10 mole % of B2O₃, 0-10 mole % of SrO. Further, in one exemplary embodiment, the zirconia layer 74 is an oxide ion conducting solid electrolyte constructed of partially yttria-doped zirconia.

The reference electrode 78 is disposed in a gap between the portions 66, 68 of the alumina layer 62 and is further disposed between the layer 60 and the zirconia layer 74. In one exemplary embodiment, the reference electrode 78 is constructed of a platinum-gold (Pt-Au) alloy.

The NO₂ sensing electrode 80 is disposed in a gap between the portions 68, 70 of the alumina layer 62 and is further disposed between the layer 60 and the zirconia layer 74. In one exemplary embodiment, the NO₂ sensing electrode is constructed of BaFe₁₂O₁₉ doped with 0-20 mole % of BaO, 0-20 mole % of (MgO, NiO, ZnO).

The combination of the NH₃ sensing electrode 76, the zirconia layer 74, and the reference electrode 78 form a NH₃ sensing cell 82 (e.g., NH₃ sensing Nernst cell) that generates a voltage indicative of a NH₃ concentration in exhaust gases communicating with the NH₃ sensing electrode 76, that is received by the computer 42.

The combination of the NO₂ sensing electrode 80, the zirconia layer 74, and the reference electrode 78 form a NO₂ sensing cell 84 (e.g., NO₂ sensing Nernst cell) that generates a voltage indicative of a NO₂ concentration in exhaust gases communicating with the NO₂ sensing electrode 80, that is received by the computer 42.

The alumina layer 90 is disposed between a second side of the zirconia layer 74 and a first side of the zirconia layer 92. A chamber 120 is formed in the alumina layer 90, which is communicated with ambient air by an air duct running through the sensing element.

The pumping electrode 94 is disposed on a first side of the zirconia layer 92 and the pumping electrode 96 is disposed on a second side of the zirconia layer 92 proximate to the pumping electrode 94. The pumping electrodes 94, 96 are further electrically coupled to the voltage source 44. In one exemplary embodiment, the pumping electrodes 94, 96 are constructed of a Pt-Au alloy.

The combination of the voltage source 44, the electrodes 94, 96 and the zirconia layer 92 comprise an electrochemical pumping cell 97. The pumping cell 97 reduces an amount of oxygen in a first portion of the chamber 122 below a first predetermined oxygen concentration level.

The O₂ reference electrode 98 is disposed on the first side of the zirconia layer 92 and the oxygen sensing electrode 100 is disposed on a second side of the zirconia layer 92 proximate to the O₂ reference electrode 98. The O₂ reference electrode 98 and the oxygen sensing electrode 100 are further electrically coupled to the computer 42. In one exemplary embodiment, the O₂ reference electrode 98 and the oxygen sensing electrode 100 are constructed of Pt. A voltage generated between the electrodes 98, 100 is indicative of an oxygen concentration in the chamber 122. The computer 42 measures the voltage between the electrodes 98, 100 indicative of the oxygen concentration, to regulate a voltage output by the voltage source 44 to maintain a concentration of oxygen in a portion of the chamber 122 below a second predetermined oxygen concentration level. The pump current driven between electrodes 94 and 96 is an indication of the oxygen concentration of the exhaust gas.

The pumping electrode 102 is disposed on a first side of the zirconia layer 92 and the pumping electrode 104 is disposed on a second side of the zirconia layer 92 proximate to the pumping electrode 102. The pumping electrodes 102, 104 are further electrically coupled to the voltage source 50. In one exemplary embodiment, the pumping electrodes 102, 104 are constructed of an Pt-Au alloy. The combination of the voltage source 50, the electrodes 102, 104 and the zirconia layer 92 comprise an electrochemical pumping cell 105. The pumping cell 105 reduces an amount of oxygen in a second portion of the chamber 122 below a second predetermined oxygen concentration level.

The alumina layer 106 is disposed between the second side of the zirconia layer 92 and a first side of the zirconia layer 108. A chamber 122 is formed in the alumina layer 106 and fluidly communicates with engine exhaust through a gas-diffusion-limiting aperture extending through a portion of the alumina layer 106. There is a second gas-diffusion-limiting aperture inside chamber 122, which separates the electrodes 96, 100 from the electrodes 104 and 110.

The zirconia layer 108 is disposed between the second side of the alumina layer 106 and a first side of the alumina layer 114. A second side of the alumina layer 114 is disposed on a first side of the alumina layer 116. A chamber 124 is formed in the alumina layer 114. Further, in one exemplary embodiment, the zirconia layer 108 is an oxide ion conducting solid electrolyte constructed of partially yttria-doped zirconia.

The NOₓ sensing electrode 110 is disposed on the first side of the zirconia layer 108 fluidly communicates with exhaust gases in the chamber 122. In one exemplary embodiment, the NOₓ sensing electrode 110 is constructed of a Pt-Rh alloy. The reference electrode 112 is disposed on the second side of the zirconia layer 108 proximate to the NOₓ sensing electrode 110 and fluidly communicates with exhaust gases in the chamber 124. In one exemplary embodiment, the reference electrode 112 is constructed of Pt alloy. The electrodes 110, 112 are electrically coupled to a series combination of the voltage source 46 and the current sensor 48.

The combination of the voltage source 46, the electrodes 110, 112 and the zirconia layer 108 comprise a NOₓ electrochemical pumping cell 113 that generates a current indicative of a NOₓ concentration in the chamber 122. The current sensor 48 outputs a signal indicative of an amount of electrical current flowing between the electrodes 110, 112, that is received by the computer 42.

The heater coil 118 is disposed in the alumina layer 116. The heater coil 118 is configured to maintain the exhaust gas sensor 40 within a predetermined temperature range in response to a control signal from the computer 42.

The temperature sensor 43 outputs a signal indicative of a temperature of the exhaust gas sensor 40 that is received by the computer 42. In one exemplary embodiment, the temperature sensor 43 is formed within the exhaust gas sensor 40 and can comprise an impedance circuit that has an impedance that varies proportional to a temperature variation. In another exemplary embodiment, the temperature sensor 43 is a stand-alone temperature sensor that is not part of the exhaust gas sensor 40.

The computer 42 is configured to calculate a NO₂ concentration value, a NH₃ concentration value, a NO concentration value, and a NOₓ concentration value indicative of: a NO₂ concentration, a NH₃ concentration, a NO concentration, and a NOₓ concentration, respectively, in exhaust gases communicating with the exhaust gas sensor 40 based on signals from the exhaust gas sensor 240 and the current sensor 48, as will be described in greater detail below. As shown, the computer 42 is electrically coupled to the voltage source 44, the NH₃ sensing electrode 76, the reference electrode 78, the NO₂ sensing electrode 80, the O₂ reference electrode 98, the oxygen sensing electrode 100, the current sensor 48, and the heater coil 118.

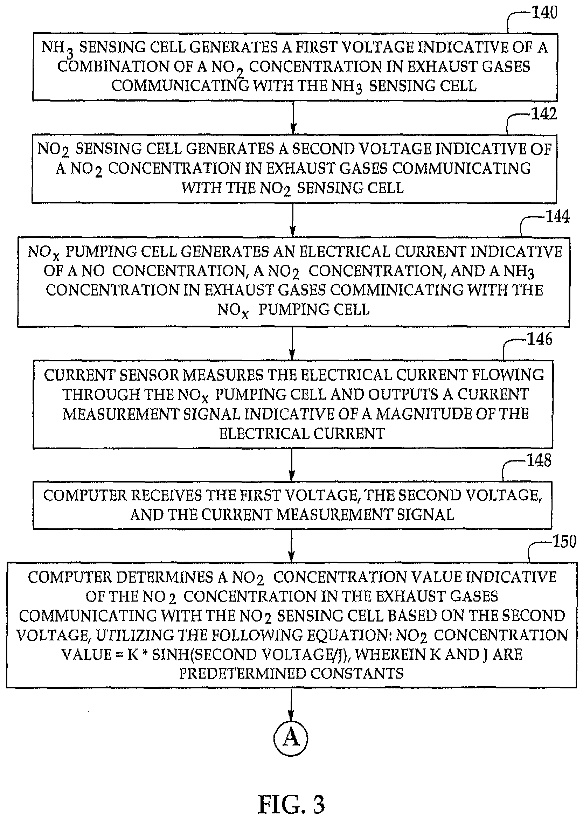

Referring to Figures 3-4, a flowchart of a method for determining concentrations of exhaust gas constituents in accordance with another exemplary embodiment will now be explained.

At step 140, the NH₃ sensing cell 82 generates a first voltage indicative of a combination of a NH₃ concentration and a NO₂ concentration in exhaust gases communicating with the NH₃ sensing cell 82.

At step 142, the NO₂ sensing cell 84 generates a second voltage indicative of a NO₂ concentration in exhaust gases communicating with the NO₂ sensing cell 84.

At step 144, the NOₓ pumping cell 113 generates an electrical current indicative of combination of a NO concentration, a NO₂ concentration, and a NH₃ concentration in exhaust gases communicating with the NOₓ pumping cell 113.

At step 146, the current sensor 48 measures the electrical current flowing through the NOₓ pumping cell 113 and outputs a current measurement signal indicative of a magnitude of the electrical current.

At step 148, the computer 42 receives the first voltage, the second voltage, and the current measurement signal.

At step 150, the computer 42 determines a NO₂ concentration value indicative of the NO₂ concentration in the exhaust gases communicating with the NO₂ sensing cell based on the second voltage, utilizing the following equation: NO₂ concentration value = K * SINH(second voltage/J), wherein K and J are predetermined constants.

At step 152, the computer 42 determines a NH₃ concentration value indicative of the NH₃ concentration in the exhaust gases communicating with the NH₃ sensing cell 82 based on the first voltage and the NO₂ concentration value, utilizing the following equation:
NH₃ concentration value = D * SINH ((first voltage - G*ASINH(NO₂ concentration value/H))/B), wherein D, G, H, and B are predetermined constants.

At step 154, the computer 42 determines a NO concentration value indicative of a NO concentration in the exhaust gases communicating with the NOₓ pumping cell 113 based on the current measurement signal, the NO₂ concentration value, and the NH₃ concentration value, utilizing the following equation:
NO concentration value = ((current measurement signal/M) - N * NO₂ concentration value - P * NH₃ concentration value), wherein M, N, and P are predetermined constants.

At step 156, the computer 42 determines a NOₓ concentration value based on the NO concentration value and the NO₂ concentration value, utilizing the following equation: NOₓ concentration value = NO concentration value + NO₂ concentration value.

At step 158, the computer 42 stores the NO₂ concentration value, the NH₃ concentration value, the NO concentration value, and the NOₓ concentration value in a memory device 43, which may be an internal memory device of the computer 42 or an external memory device to the computer 40.

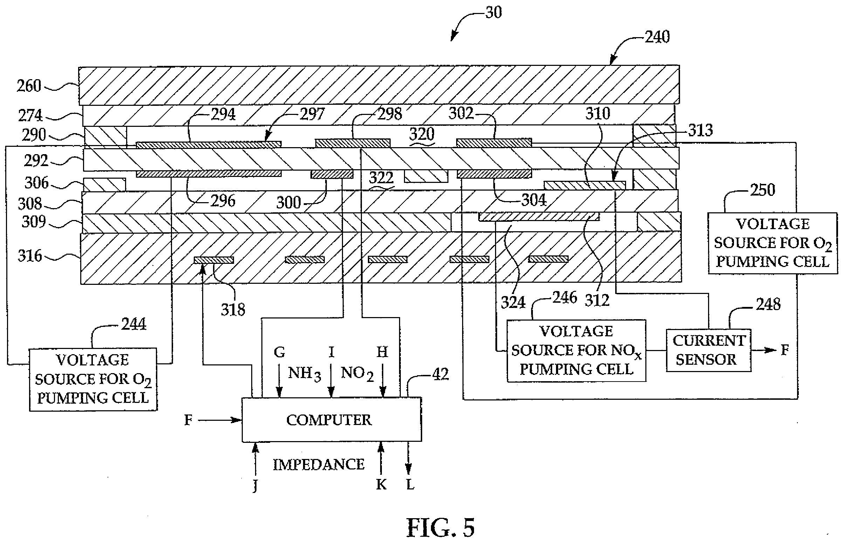

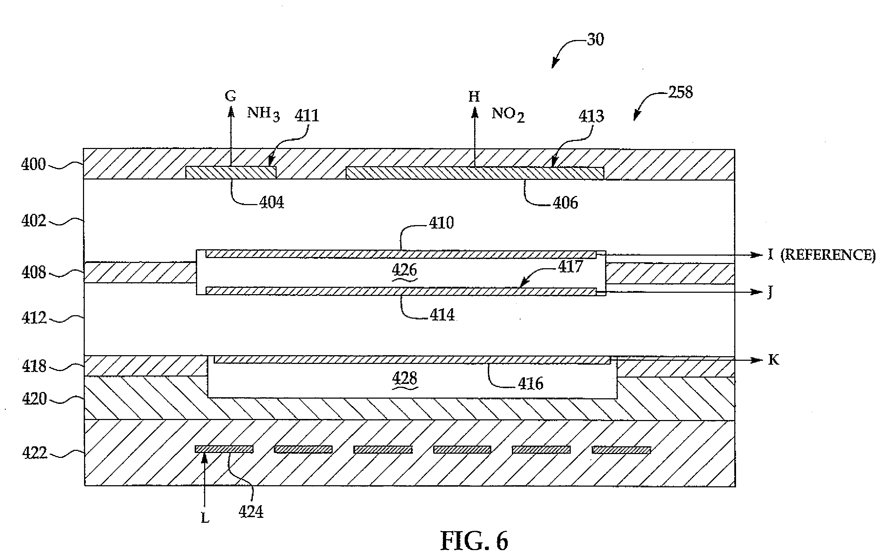

Referring to Figures 5 and 6, the exhaust gas sensing system 30 can be utilized instead of the exhaust gas sensing system 28 to determine concentrations of NO₂, NH₃, NO, and NOₓ in the exhaust gases. The exhaust gas sensing system 30 includes an exhaust gas sensor 240, the computer 42, a voltage source 244, a voltage source 246, a current sensor 248, a voltage source 250, and an exhaust gas sensor 258.

The exhaust gas sensor 240 communicates with the exhaust gases in the exhaust pipe 26 and is configured to generate signals that are indicative of a NOₓ concentration in the exhaust gases. The exhaust gas sensor 240 includes a porous protection layer 260, a zirconia layer 274, an alumina layer 290, a zirconia layer 292, pump electrodes 294, 296, an O₂ sensing electrode 300, a reference electrode 298, pump electrodes 302, 304, an alumina layer 306, a zirconia layer 308, a NOₓ sensing electrode 310, a reference electrode 312, alumina layers 309, 316, and a heater coil 318.

The porous protection layer 260 is configured to protect the zirconia layer 274. In one exemplary embodiment, the porous protection layer 260 is constructed from porous alumina. As shown, the layer 260 is disposed at least in part on a first side of the zirconia layer 274. In one exemplary embodiment, the zirconia layer 274 is an oxide ion conducting solid electrolyte constructed of partially yttria-doped zirconia.

The alumina layer 290 is disposed between a second side of the zirconia layer 274 and a first side of the zirconia layer 292. A chamber 320 is formed in the alumina layer 290, which is communicated with ambient air by an air duct running through the sensing element body.

The pumping electrode 294 is disposed on a first side of the zirconia layer 292 and the pumping electrode 296 is disposed on a second side of the zirconia layer 292 proximate to the pumping electrode 294. The pumping electrodes 294, 296 are further electrically coupled to the voltage source 244. In one exemplary embodiment, the pumping electrodes 294, 296 are constructed of a Pt-Au alloy.

The combination of the voltage source 244, the electrodes 294, 296 and the zirconia layer 292 comprise an electrochemical pumping cell 297. The pumping cell 296 reduces an amount of oxygen in a first portion of the chamber 322 below a first predetermined oxygen concentration level.

The O₂ sensing electrode 298 is disposed on the first side of the zirconia layer 292 and the oxygen sensing electrode 300 is disposed on a second side of the zirconia layer 292 proximate to the O₂ reference electrode 298. The O₂ sensing electrode 300 and the reference electrode 298 are further electrically coupled to the computer 42. In one exemplary embodiment, the O₂ sensing electrode 300 and the reference electrode 298 are constructed of Pt. A voltage generated between the electrodes 298, 300 is indicative of an oxygen concentration in the chamber 322. The computer 42 measures the voltage between the electrodes 298, 300, indicative of the oxygen concentration, to regulate a voltage output by the voltage source 244 to maintain a concentration of oxygen in a portion of the chamber 322 below a second predetermined oxygen concentration level.

The pumping electrode 302 is disposed on a first side of the zirconia layer 292 and the pumping electrode 304 is disposed on a second side of the zirconia layer 292 proximate to the pumping electrode 302. The pumping electrodes 302, 304 are further electrically coupled to the voltage source 250. In one exemplary embodiment, the pumping electrodes 302, 304 are constructed of an Pt-Au alloy. The combination of the voltage source 250, the electrodes 302, 304 and the zirconia layer 292 comprise an electrochemical pumping cell 297. The pumping cell 297 reduces an amount of oxygen in a second portion of the chamber 322 below a second predetermined oxygen concentration level.

The alumina layer 306 is disposed between the second side of the zirconia layer 292 and a first side of the zirconia layer 308. A chamber 322 is formed in the alumina layer 306 and fluidly communicates with engine exhaust with a gas-diffusion-limiting aperture extending through a portion of the alumina layer 306. A second gas-diffusion-limiting aperture is set between the electrode 300 and the electrode 304 to limit the gas diffusion within the chamber 322 between the electrodes 300 and 304.

The zirconia layer 308 is disposed between the second side of the alumina layer 306 and a first side of the alumina layer 309. A second side of the alumina layer 309 is disposed on a first side of the alumina layer 316. A chamber 324 is formed in the alumina layer 309. Further, in one exemplary embodiment, the zirconia layer 308 is an oxide ion conducting solid electrolyte constructed of partially yttria-doped zirconia.

The NOₓ sensing electrode 310 is disposed on the first side of the zirconia layer 308 fluidly communicates with exhaust gases in the chamber 322. In one exemplary embodiment, the NOₓ sensing electrode 310 is constructed of a Pt-Rh alloy. The reference electrode 312 is disposed on the second side of the zirconia layer 308 proximate to the NOₓ sensing electrode 310 and fluidly communicates with exhaust gases in the chamber 324. In one exemplary embodiment, the reference electrode 312 is constructed of Pt-Au alloy. The electrodes 310, 312 are electrically coupled to a series combination of the voltage source 246 and a current sensor 248.

The combination of the voltage source 246, the electrodes 310, 312 and the zirconia layer 308 comprise a NOₓ electrochemical pumping cell 313 that generates a current indicative of a NOₓ concentration in the chamber 322. The current sensor 248 outputs a signal indicative of an amount of electrical current flowing between the electrodes 310, 312, that is received by the computer 42.

The heater coil 318 is disposed in the alumina layer 316. The heater coil 318 is configured to maintain the exhaust gas sensor 240 within a predetermined temperature range in response to a control signal from the computer 42.

Referring to Figure 6, the exhaust gas sensor 258 will now be explained. The exhaust gas sensor 258 communicates with the exhaust gases in the exhaust pipe 26 and is configured to generate signals indicative of a NH₃ concentration and a NO₂ concentration, and a temperature of the exhaust gas sensor 258.

The exhaust gas sensor 258 includes a porous protection layer 400, a zirconia layer 402, a NH₃ sensing electrode 404, a NO₂ sensing electrode 406, a porous alumina layer 408, a reference electrode 410, a zirconia layer 412, reference electrodes 414, 416, a porous alumina layer 418, alumina layers 420, 422, and a heater coil 424.

The porous protection layer 400 is configured to allow exhaust gases to diffuse therethrough to contact the NH₃ sensing electrode 404 and the NO₂ sensing electrode 406. As shown, the layer 400 is disposed at least in part on a first side of the zirconia layer 402. In one exemplary embodiment, the porous protection layer 400 is constructed from porous alumina, and the zirconia layer 402 is an oxide ion conducting solid electrolyte constructed of partially yttria-doped zirconia. Further, the NH₃ sensing electrode 404 is constructed of BiVO₄ doped with 0-20 mole % of MgO, 0-10 mole % of B2O₃, 0-10 mole % of SrO and the NO₂ sensing electrode is constructed of BaFe₁₂O₁₉ doped with 0-20 mole % of BaO, 0-20 mole % of (MgO, NiO, ZnO). The reference electrode 410 is disposed on a second side of the zirconia layer 410. In one exemplary embodiment, the reference electrode 410 is constructed of a Pt alloy.

The combination of the NH₃ sensing electrode 404, the zirconia layer 402, and the reference electrode 410 form a NH₃ sensing cell 411 (e.g., NH₃ sensing Nernst cell) that generates a voltage indicative of a NH₃ concentration communicating with the NH₃ sensing electrode 404, that is received by the computer 42.

The combination of the NO₂ sensing electrode 406, the zirconia layer 402, and the reference electrode 410 form a NO₂ sensing cell 413 (e.g., NO₂ sensing Nernst cell) that generates a voltage indicative of a NO₂ concentration communicating with the NO₂ sensing electrode 406, that is received by the computer 42.

A portion of a second side of the zirconia layer 402 is disposed on a first side of the alumina layer 408. A chamber 426 is formed in the alumina layer 408 and adjacent portions of the zirconia layer 402 and the zirconia layer 412. At least a peripheral portion of the alumina layer 408 is porous which allows exhaust gases to diffuse therethrough into the chamber 426. A reference electrode 414 is disposed on a first side of the zirconia layer 412 and communicates with exhaust gases in the chamber 426.

A portion of a second side of the alumina layer 408 is disposed on a first side of the zirconia layer 412. A portion of a second side of the zirconia layer 412 is disposed on a first side of the alumina layer 418. A chamber 428 is formed in the alumina layer 418 and a portion of the alumina layer 420. The reference electrode 416 is disposed on the second side of the zirconia layer 412. The chamber 428 communicates with the engine exhaust gases via a portion of the alumina layer 418 being constructed of gas porous alumina.

The combination of the electrodes 414, 416 and the zirconia layer 412 comprise a temperature sensor 417. In particular, an impedance between the electrodes 414, 416 is indicative of a temperature of the exhaust gas sensor 258 and a temperature of exhaust gases communicating with the exhaust gas sensor 258. The computer 42 can measure the impedance between the electrodes 414, 416 and determine a temperature value indicative of the temperature of the sensor 258, based on the impedance.

A second side of the alumina layer 420 is disposed on the alumina layer 422. The heater coil 424 is disposed in the alumina layer 420. The heater coil 424 is configured to maintain the exhaust gas sensor 258 within a predetermined temperature range in response to a control signal from the computer 42.

In an alternative embodiment, the computer 42 is configured to calculate a NO₂ concentration value, a NH₃ concentration value, a NO concentration value, and a NOₓ concentration value indicative of: a NO₂ concentration, a NH₃ concentration, a NO concentration, and a NOₓ concentration, respectively, in exhaust gases communicating with the exhaust gas sensors 240, 258 based on signals from the sensors 240, 258, and the current sensor 248. As shown, the computer 42 is electrically coupled to the voltage source 244, the NH₃ sensing electrode 404, the reference electrode 410, the NO₂ sensing electrode 406, the current sensor 248, and the heater coil 424.

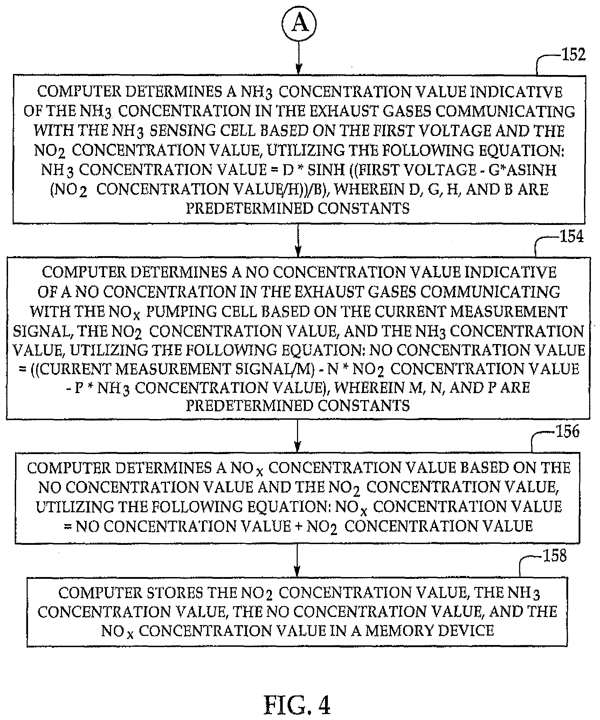

Further, it should be noted that the exhaust gas sensing system 30 can be utilized to implement the method described in the flowchart of Figures 3 and 4, instead of the exhaust gas sensing system 28, utilizing the signals from the sensors 240, 258, and the current sensor 248.

The exhaust gas sensing systems and the method for determining concentrations of exhaust gas constituents provide a substantial advantage over other systems and methods. In particular, the exhaust gas sensing systems and the method provide a technical effect of accurately determining NOₓ, NO₂, NH₃, and NO concentrations in exhaust gases.

While embodiments of the invention are described with reference to the exemplary embodiments, it will be understood by those skilled in the art that various changes may be made and equivalents may be substituted for elements thereof without departing from the scope of the invention. In addition, many modifications may be made to the teachings of the invention to adapt to a particular situation without departing from the scope thereof. Therefore, it is intended that the invention not be limited to the embodiment disclosed for carrying out this invention, but that the invention includes all embodiments falling within the scope of the intended claims. Moreover, the use of the terms first, second, etc. does not denote any order of importance, but rather the terms first, second, etc. are used to distinguish one element from another. Furthermore, the use of the terms a, an, etc. do not denote a limitation of quantity, but rather denote the presence of at least one of the referenced items.

## Claims

1. An exhaust gas sensing system (28), comprising:
a NH₃ sensing cell (82) configured to generate a first voltage indicative of a combination of a NH₃ concentration and a NO₂ concentration in exhaust gases communicating with the NH₃ sensing cell (82);
a NO₂ sensing cell (84) configured to generate a second voltage indicative of a NO₂ concentration in exhaust gases communicating with the NO₂ sensing cell (84);
a NOₓ pumping cell (113) configured to generate an electrical current indicative of combination of a NO concentration, a NO₂ concentration, and a NH₃ concentration in exhaust gases communicating with the NOₓ pumping cell (113);
a current sensor (48) configured to measure the electrical current flowing through the NOₓ pumping cell (113) and to output a current measurement signal indicative of a magnitude of the electrical current;
a computer (42) configured to receive the first voltage, the second voltage, and the current measurement signal, the computer further configured to determine a NO₂ concentration value indicative of the NO₂ concentration in the exhaust gases communicating with the NO₂ sensing cell (84) based on the second voltage;
the computer further configured to determine a NH₃ concentration value indicative of the NH₃ concentration in the exhaust gases communicating with the NH3 sensing cell (82) based on the first voltage and the NO₂ concentration value;
the computer further configured to determine a NO concentration value indicative of a NO concentration in the exhaust gases communicating with the NOx pumping cell (113) based on the current measurement signal, the NO₂ concentration value, and the NH₃ concentration value;
the computer further configured to determine a NOₓ concentration value based on the NO concentration value and the NO₂ concentration value; and
the computer (42) further configured to store the NO₂ concentration value, the NH₃ concentration value, the NO concentration value, and the NOₓ concentration value in a memory device.

2. The exhaust gas sensing system (30) of claim 1, wherein the NH3 sensing cell (411) and the NO₂ sensing cell (413) are disposed in a first exhaust gas sensor (258) and the NOx pumping cell (313) is disposed in a second exhaust gas sensor (240) that is not a part of the first exhaust gas sensor.

3. The exhaust gas sensing system (28) of claim 1, wherein the NH₃ sensing cell (82), the NO2 sensing cell (84), and the NOx pumping cell (113) are disposed in an exhaust gas sensor (40).

4. A method (140-158) for determining concentrations of exhaust gas constituents, comprising:
generating a first voltage utilizing a NH3 sensing cell (82,411) that is indicative of a combination of a NH₃ concentration and a NO₂ concentration in exhaust gases communicating with the NH3 sensing cell (82,411) ;
generating a second voltage utilizing a NO2 sensing cell (84, 413) that is indicative of a NO₂ concentration in exhaust gases communicating with the NO2 sensing cell (84, 413);
generating an electrical current utilizing a NOx pumping cell (113, 313) that is indicative of combination of a NO concentration, a NO₂ concentration, and a NH₃ concentration in exhaust gases communicating with the NOx pumping cell (113, 313);
measuring the electrical current flowing through the NOx pumping cell (113, 313) utilizing a current sensor (48, 248) and outputting a current measurement signal from the current sensor (48, 248) indicative of a magnitude of the electrical current;
receiving the first voltage, the second voltage, and the current measurement signal at a computer (42);
determining a NO₂ concentration value indicative of the NO₂ concentration in the exhaust gases communicating with the NO2 sensing cell (84, 413) based on the second voltage, utilizing the computer;
determining a NH₃ concentration value indicative of the NH₃ concentration in the exhaust gases communicating with the NH3 sensing cell (82,411) based on the first voltage and the NO₂ concentration value, utilizing the computer;
determining a NO concentration value indicative of a NO concentration in the exhaust gases communicating with the NOx pumping cell (113, 313) based on the current measurement signal, the NO₂ concentration value, and the NH₃ concentration value, utilizing the computer;
determining a NOₓ concentration value based on the NO concentration value and the NO₂ concentration value, utilizing the computer; and
storing the NO₂ concentration value, the NH₃ concentration value, the NO concentration value, and the NOₓ concentration value in a memory device, utilizing the computer.

## Patentansprüche

1. Abgaserfassungssystem (28), das aufweist:
eine NH₃-Erfassungszelle (82), die konfiguriert ist zum Erzeugen einer ersten Spannung, die eine Kombination einer NH₃-Konzentration und einer NO₂-Konzentration in Abgasen angibt, die mit der NH₃-Erfassungszelle (82) in Verbindung sind;
eine NO₂-Erfassungszelle (84), die konfiguriert ist zum Erzeugen einer zweiten Spannung, die eine NO₂-Konzentration in Abgasen angibt, die mit der NO₂-Erfassungszelle (84) in Verbindung sind;
eine NOₓ-Pumpzelle (113), die konfiguriert ist zum Erzeugen eines elektrischen Stroms, der eine Kombination einer NO-Konzentration, einer NO₂-Konzentration und einer NH₃-Konzentration in Abgasen angibt, die mit der NOₓ-Pumpzelle (113) in Verbindung sind;
einen Stromsensor (48), der konfiguriert ist zum Messen des elektrischen Stroms, der durch die NOₓ-Pumpzelle (113) fließt, und zum Ausgeben eines Strommesssignals, das eine Größe des elektrischen Stroms angibt;
einen Computer (42), der konfiguriert ist zum Empfangen der ersten Spannung, der zweiten Spannung und des Strommesssignals, wobei der Computer weiter konfiguriert ist zum Bestimmen eines NO₂-Konzentrationswerts, der die NO₂-Konzentration in den Abgasen angibt, die mit der NO₂-Erfassungszelle (84) in Verbindung sind, basierend auf der zweiten Spannung;
der Computer weiter konfiguriert ist zum Bestimmen eines NH₃-Konzentrationswerts, der die NH₃-Konzentration in den Abgasen angibt, die mit der NH₃-Erfassungszelle (82) in Verbindung sind, basierend auf der ersten Spannung und dem NO₂-Konzentrationswert;
der Computer weiter konfiguriert ist zum Bestimmen eines NO-Konzentrationswerts, der eine NO-Konzentration in den Abgasen angibt, die mit der NOₓ-Pumpzelle (113) in Verbindung sind, basierend auf dem Strommesssignal, dem NO₂-Konzentrationswert und dem NH₃-Konzentrationswert;
der Computer weiter konfiguriert ist zum Bestimmen eines NOₓ-Konzentrationswerts basierend auf dem NO-Konzentrationswert und dem NO₂-Konzentrationswert; und
der Computer (42) weiter konfiguriert ist zum Speichern des NO₂-Konzentrationswerts, des NH₃-Konzentrationswerts, des NO-Konzentrationswerts und des NOₓ-Konzentrationswerts in einer Speichervorrichtung.

2. Das Abgaserfassungssystem (30) gemäß Anspruch 1, wobei die NH₃-Erfassungszelle (411) und die NO₂-Erfassungszelle (413) in einem ersten Abgassensor (258) angeordnet sind und die NOₓ-Pumpzelle (313) in einem zweiten Abgassensor (240) angeordnet ist, der nicht Teil des ersten Abgassensors ist.

3. Das Abgaserfassungssystem (28) gemäß Anspruch 1, wobei die NH₃-Erfassungszelle (82), die NO₂-Erfassungszelle (84) und die NOₓ-Pumpzelle (113) in einem Abgassensor (40) angeordnet sind.

4. Ein Verfahren (140-158) zum Bestimmen von Konzentrationen von Abgasbestandteilen, das aufweist:
Erzeugen einer ersten Spannung unter Verwendung einer NH₃-Erfassungszelle (82, 411), die eine Kombination einer NH₃-Konzentration und einer NO₂-Konzentration in Abgasen angibt, die mit der NH₃-Erfassungszelle (82, 411) in Verbindung sind;
Erzeugen einer zweiten Spannung unter Verwendung einer NO₂-Erfassungszelle (84, 413), die eine NO₂-Konzentration in Abgasen angibt, die mit der NO₂-Erfassungszelle (84, 413) in Verbindung sind;
Erzeugen eines elektrischen Stroms unter Verwendung einer NOₓ-Pumpzelle (113, 313), die eine Kombination einer NO-Konzentration, einer NO₂-Konzentration und einer NH₃-Konzentration in Abgasen angibt, die mit der NOₓ-Pumpzelle (113, 313) in Verbindung sind;
Messen des elektrischen Stroms, der durch die NOₓ-Pumpzelle (113, 313) fließt, unter Verwendung eines Stromsensors (48, 248) und Ausgeben eines Strommesssignals von dem Stromsensor (48, 248), das eine Größe des elektrischen Stroms angibt;
Empfangen der ersten Spannung, der zweiten Spannung und des Strommesssignals an einem Computer (42);
Bestimmen eines NO₂-Konzentrationswerts, der die NO₂-Konzentration in den Abgasen angibt, die mit der N02-Erfassungszelle (84, 413) in Verbindung sind, basierend auf der zweiten Spannung, unter Verwendung des Computers;
Bestimmen eines NH₃-Konzentrationswerts, der die NH₃-Konzentration in den Abgasen angibt, die mit der NH₃-Erfassungszelle (82, 411) in Verbindung sind, basieren auf der ersten Spannung und dem NO₂-Konzentrationswert, unter Verwendung des Computers;
Bestimmen eines NO-Konzentrationswerts, der eine NO-Konzentration in den Abgasen angibt, die mit der NOₓ-Pumpzelle (113, 313) in Verbindung sind, basierend auf dem Strommesssignal, dem NO₂-Konzentrationswert und dem NH₃-Konzentrationswert, unter Verwendung des Computers;
Bestimmen eines NOₓ-Konzentrationswerts basierend auf dem NO-Konzentrationswert und dem NO₂-Konzentrationswert, unter Verwendung des Computers; und
Speichern des NO₂-Konzentrationswerts, des NH₃-Konzentrationswerts, des NO-Konzentrationswerts und des NOₓ-Konzentrationswerts in einer Speichervorrichtung, unter Verwendung des Computers.

## Revendications

1. Système de détection de gaz d'échappement (28), comprenant :
une cellule de détection de NH3 (82) configurée pour générer une première tension indicative d'une combinaison d'une concentration en NH3 et d'une concentration en NO2 dans les gaz d'échappement qui communiquent avec la cellule de détection de NH3 (82) ;
une cellule de détection de NO2 (84) configurée pour générer une seconde tension indicative d'une concentration en NO2 dans les gaz d'échappement qui communiquent avec la cellule de détection de NO2 (84) ;
une cellule de pompage de NOx (113) configurée pour générer un courant électrique indicatif d'une combinaison d'une concentration en NO, d'une concentration en NO2 et d'une concentration en NH3 dans les gaz d'échappement qui communiquent avec la cellule de pompage de NOx (113) ;
un détecteur de courant (48) configuré pour mesurer le courant électrique qui s'écoule à travers la cellule de pompage de NOx (113) et pour délivrer un signal de mesure de courant indicatif d'une intensité du courant électrique ;
un ordinateur (42) configuré pour recevoir la première tension, la seconde tension, et le signal de mesure de courant, l'ordinateur étant en outre configuré pour déterminer une valeur de concentration de NO2 indicative de la concentration en NO2 dans les gaz d'échappement qui communiquent avec la cellule de détection de NO2 (84) sur la base de la seconde tension ;
l'ordinateur étant en outre configuré pour déterminer une valeur de concentration de NH3 indicative de la concentration en NH3 dans les gaz d'échappement qui communiquent avec la cellule de détection de NH3 (82) sur la base de la première tension et de la valeur de concentration en NO2 ;
l'ordinateur étant en outre configuré pour déterminer une valeur de concentration en NO indicative d'une concentration en NO dans les gaz d'échappement qui communiquent avec la cellule de pompage de NOx (113) sur la base du signal de mesure de courant, de la valeur de concentration en NO2, et de la valeur de concentration en NH3 ;
l'ordinateur étant en outre configuré pour déterminer une valeur de concentration en NOx sur la base de la valeur de concentration en NO et de la valeur de concentration en NO2 ; et
l'ordinateur (42) étant en outre configuré pour stocker la valeur de concentration en NO2, la valeur de concentration en NH3, la valeur de concentration en NO, et la valeur de concentration en NOx dans un dispositif à mémoire.

2. Système de détection de gaz d'échappement (30) selon la revendication 1, dans lequel la cellule de détection de NH3 (411) et la cellule de détection de NO2 (413) sont disposées dans un premier détecteur de gaz d'échappement (258) et la cellule de pompage de NOx (313) est disposée dans un second détecteur de gaz d'échappement (240) qui ne fait pas partie du premier détecteur de gaz d'échappement.

3. Système de détection de gaz d'échappement (28) selon la revendication 1, dans lequel la cellule de détection de NH3 (82), la cellule de détection de NO2 (84), et la cellule de pompage de NOx (113) sont disposées dans un détecteur de gaz d'échappement (40).

4. Procédé (140-158) pour déterminer les concentrations de constituants d'un gaz d'échappement, comprenant les étapes consistant à :
générer une première tension en utilisant une cellule de détection de NH3 (82, 411) qui est indicative d'une combinaison d'une concentration en NH3 et d'une concentration en NO2 dans les gaz d'échappement qui communiquent avec la cellule de détection de NH3 (82, 411) ;
générer une seconde tension en utilisant une cellule de détection de NO2 (84, 413) qui est indicative d'une concentration en NO2 dans les gaz d'échappement qui communiquent avec la cellule de détection de NO2 (84, 413) ;
générer un courant électrique en utilisant une cellule de pompage de NOx (113, 313) qui est indicative d'une combinaison d'une concentration en NO, d'une concentration en NO2, et d'une concentration en NH3 dans les gaz d'échappement qui communiquent avec la cellule de pompage de NOx (113, 313) ;
mesurer le courant électrique qui s'écoule à travers la cellule de pompage de NOx (113, 313) en utilisant un détecteur de courant (48, 248) et en délivrant un signal de mesure de courant depuis le détecteur de courant (48, 248), indicatif d'une intensité du courant électrique ;
recevoir la première tension, la seconde tension, et le signal de mesure de courant dans un ordinateur (42) ;
déterminer une valeur de concentration en NO2 indicative de la concentration en NO2 dans les gaz d'échappement qui communiquent avec la cellule de détection de NO2 (84, 413) sur la base de la seconde tension, en utilisant l'ordinateur ;
déterminer une valeur de concentration en NH3 indicative de la concentration en NH3 dans les gaz d'échappement qui communiquent avec la cellule de détection de NH3 (82, 411) sur la base de la première tension et de la valeur de concentration en NO2, en utilisant l'ordinateur ;
déterminer une valeur de concentration en NO indicative d'une concentration en NO dans les gaz d'échappement qui communiquent avec la cellule de pompage de NOx (113, 313) sur la base du signal de mesure de courant, de la valeur de concentration en NO2, et de la valeur de concentration en NH3, en utilisant l'ordinateur ;
déterminer une valeur de concentration en NOx sur la base de la valeur de concentration en NO et de la valeur de concentration en NO2, en utilisant l'ordinateur ; et
stocker la valeur de concentration en NO2, la valeur de concentration en NH3, la valeur de concentration en NO, et la valeur de concentration en NOx dans un dispositif à mémoire, en utilisant l'ordinateur.
